# EUROPEAN PATENT APPLICATION

(11) **EP 1 096 256 A1**
(43) Date of publication of application: **02.05.2001**
(21) Application number: 00925606.6
(22) Date of filing: 11.05.2000
(51) Int. Cl.: G01N 33/543

(54) **CHROMATOGRAPHY QUANTITATIVE MEASUREMENT DEVICE, CHROMATOGRAPHY QUANTITATIVE MEASUREMENT METHOD, AND CHROMATOGRAPHY TEST PIECE USED THEREFOR**

(30) Priority: 13.05.1999 JP 13243299
(71) Applicant: MATSUSHITA ELECTRIC INDUSTRIAL CO., LTD., Kadoma-shi, Osaka 571-8501 (JP)
(72) Inventor: TAKAHASHI, Mie, Niihama-shi, Ehime 792-0026 (JP); NADAOKA, Masataka, Iyo-shi, Ehime 799-3113 (JP); TANAKA, Hirotaka, Matsuyama-shi, Ehime 791-1102 (JP)
(74) Representative: Kügele, Bernhard
(86) International application number: JP0003002
(87) International publication number: WO0070346

(57) **Abstract**

The technologies disclosed by the present invention allow quantitative measurement to be conducted accurately on the concentration of at least one analyte contained in liquid sample, includes: a chromatographic strip (10) with a plurality of reaction areas (6A, 6B) retaining a binding reagent to make an specific reaction with the analytes, thereby making it possible for the binding reagent to take on a coloration; and a coloration level measuring means (11) to carry out quantitative measurement with a numerical expression on the coloration level of at least two or more of reaction areas out of the plurality of reaction areas. The coloration level measuring means has a capability of conducting measurement of the extent of a coloration occurring as a result of a reaction between the binding reagent and the analytes. The measurement result is subjected to computation processing in a computation processing device (12), thereby deriving by computation the concentration of the analytes in a numerical expression.

## Description

### Technical Field

The present invention relates to a chromatographic quantitative measurement apparatus, a method of chromatographic quantitative measurement and a chromatographic strip for use in chromatographic quantitative measurement for the purpose of making quantitative measurements on a substance to be tested through the use of an antigen-antibody reaction.

### Background Art

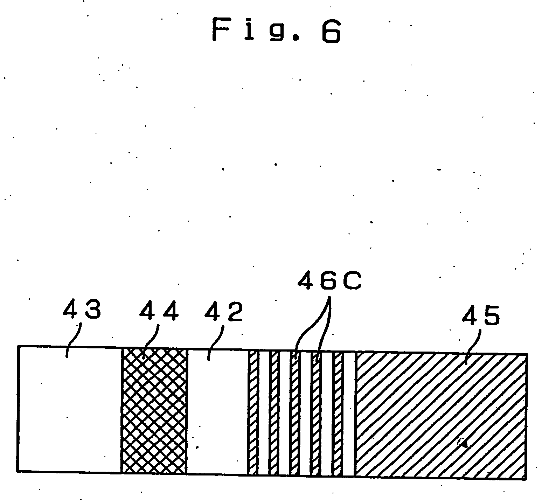

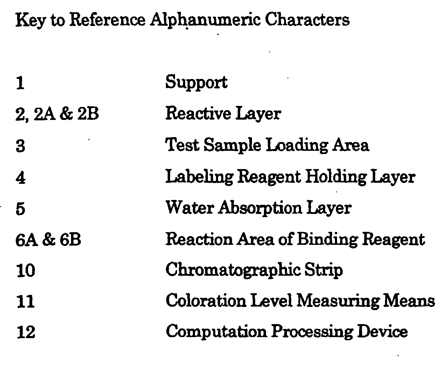

As an apparatus for carrying out a chemical test or pre-market test of liquid sample serving as a substance to be tested, a measurement apparatus featuring an application of immuno-chromatographic utilizing an antigen-antibody reaction has been widely in use. Conventional measurements using an immuno-chromatographic strip have been performed by a semiquantitative measurement method. A description is given to an example of the conventional measurement method. Fig. 6 is a plan view to show the structure of a conventional immuno-chromatographic strip. As Fig. 6 shows, the conventional immuno-chromatographic strip is provided with a test sample loading area 43, a labeling reagent holding layer 44, at least one reactive layer 42 and a water absorption layer 45. Liquid sample is allowed to move through these layers. The labeling reagent holding layer 44 holds a labeling reagent which is made movable by having liquid sample infiltrated therein. At least one reactive layer 42 has a binding reagent immobilized therein, the binding reagent exercising a function to carry out specific bond reactions with at least one analyte that has drifted thereto. At least one reactive layer 42 has a plurality of reaction areas 46C formed of a predetermined number of binding reagent immobilized sections, each being shaped like a linear strip. The water absorption layer 45 exercises a function to absorb water from a test sample that has drifted thereto.

The immuno-chromatographic strip of above is referred to as a reaction layer carrier. A liquid sample is added to the reaction layer carrier and left standing for a fixed period of time. By checking a coloration reaction that appears after a fixed period of time, whether a substance to be checked exists or not is determined. More specifically, when the liquid sample is added to the test sample loading area 43, the liquid sample infiltrates in the test sample loading area 43 and reaches the labeling reagent holding layer 44. The labeling reagent is resolved in the labeling reagent holding layer 44 as the liquid sample infiltrates therein, resulting in infiltration of the labeling reagent into the at least one reactive layer 42. During the foregoing process, the analytes contained in the liquid sample and the labeling reagent are bonded together to produce complex. These complex causes specific reactions with the binding reagent of the binding reagent immobilized section in the linear strip structured reaction areas 46C, thereby bringing about a coloration reaction.

When the quantity of the analytes contained in the liquid sample is small, the quantity of the labeling reagent complex becomes also small. As a result, the areas where the coloration reaction takes place in the at least one reactive layer 42 are limited to an upstream area of the reaction areas 46C formed of a plurality of linear strip-like shapes, thereby ending up with a smaller number of linear strips where a coloration reaction takes place. On the other hand, when the content of the analytes is large in quantity, the quantity of the labeling reagent complex becomes also large, resulting in having the area, where the coloration reaction takes place, extended to a downstream area of the reaction areas 46C formed of the plurality of linear strip-like shapes.

Conventionally, according to the foregoing principle, checking the number of the strips of the reaction areas 46C, where the coloration reaction takes place, has allowed the concentration of the analytes contained in the liquid sample to be determined semiquantitatively on a scale from one to three, from one to five, from one to ten and the like between "large" and "small".

A method for quantifying the analysis result of immuno-chromatographic according to the method as above is disclosed in the Japanese Patent Unexamined Publication No. H5-5743, for example. According to the conventional technology as disclosed in above, an independently immobilized binding reagent is dispersed to a plurality of reaction area locations on a chromatographic strip in such a way as the concentration of at least one analyte increases successively from upstream to downstream of the chromatographic strip. By counting visually the number of reaction areas where a coloration is occurs at the plurality of reaction area locations dispersed with the binding reagent, semiquantitative measurements of the analytes can be conducted.

Another conventional technology is disclosed in the Japanese Patent Unexamined Publication No. H6-341989. According to this conventional technology, an antibody against at least one analyte that exists in a predetermined immobilized quantity allows a fixed quantity of the analytes in test sample corresponding to the quantity of the immobilized antibody to be bound, thereby having the analytical object reduced in quantity. Accordingly, semiquantification of the analytes is facilitated.

Still another conventional technology is disclosed in the Japanese Patent Unexamined Publication No. H8-240591. According to this conventional technology, test sample is added to an immuno-chromatographic strip and a reaction takes place. Then, an extent of coloration is measured in a predetermined detection area. In other words, such signals as absorption, reflection and the like of the area where the coloration reaction takes place are measured by means of a measurement instrument for detection, thereby allowing the quantification of measurement results to be carried out.

The measurement principle of immuno-chromatographic allows the specific characteristics of an antigen-antibody reaction to be utilized and also can be used in examining a variety of objects to be analyzed. However, The conventional immuno-chromatographic measurement as described in above just remains as semiquantitative measurement relying on the kinds of objects to be analyzed and makes quantitative measurements impossible, thus setting a limit of allowing only semiquantitative measurements to be performed according to the kinds of objects to be analyzed. Therefore, it has been long desired to realize a quantitative measurement apparatus for obtaining an accurate analysis result by the use of immuno-chromatographic strips.

When quantitative measurements are conducted by the use of an immuno-chromatographic strip, it is conceivable that a method for measuring the concentration of at least one analyte based on the degree of coloration level or that of light and shade is to be used. When a conventional immuno-chromatographic strip is used, the development of a chromatographic application has been made under the condition where no mechanical control is put in place, hence not allowing the reaction speed to be controlled artificially. Therefore, the osmotic speed one analytes is dependent on the osmotic ability of the test strip itself. More specifically, the osmotic speed is dependent on the timing of measurement, the kind of a test strip, positions on the test strip, the quantity of liquid sample, the quantity of a labeling reagent and the like, resulting in a wide range of variation in coloration level on the test strip. When the reaction area is limited to a specific place in location and the degree of a coloration level at the reaction area limited to the specific place in location is measured, a resulting measurement error tends to be enlarged due to the osmotic ability of a labeling reagent as employed, thereby making it difficult for the concentration of analyte to be measured quantitatively with a numerical expression. As a result, the performance of quantitative measurement according to immuno-chromatographic has been ending up with close to semiquantitative measurement.

The present invention deals with realization of a chromatographic quantitative measurement apparatus, a chromatographic quantitative measurement method and a chromatographic strip, whereby the quantitative measurement performance in immuno-chromatographic is allowed to be enhanced.

### Summary of the Invention

A chromatographic quantitative measurement apparatus according to the present invention, whereby the concentration, of at least one analyte contained in liquid sample is measured, comprises:
a chromatographic strip having a plurality of reaction areas, each of the plurality of reactive areas holding a binding reagent that can take on a coloration by forming specific bond with the analytes; and
a coloration level measuring means to measure quantitatively with a numerical expression the coloration level of reaction area at least two or more areas out of the plurality of reaction areas,
wherein the coloration level measuring means has the function of conducting at least one of an optical measurement or an image measurement of the extent of a coloration which specific bond between the binding reagent and the analytes takes on.

Particularly preferred, the chromatographic quantitative measurement apparatus further comprises a computation-processing device for subjecting a measurement result of the coloration level to a computation process to derive by computation the concentration of the analytes by in number.

A chromatographic quantitative measurement method of the present invention for measuring the concentration of at least one analyte contained in liquid sample comprises the steps of:
(a) providing a chromatographic strip having a plurality of reaction areas immobilizing binding reagent that is allowed to bond with substance containing the analytes;
(b) moistening the chromatographic strip with the liquid sample containing the analytes, and bringing the substance containing the analytes into contact with the binding reagent ; and
(c) measuring the condition of a coloration in at least two reaction areas of the plurality of reaction areas, the coloration being taken on by an specific bond between the binding reagent and the analytes by way of at least one of the optical measurement or image measurement, and deriving by computation the concentration by a numerical expression via a computation process applied to the measurement result.

A chromatographic strip of the present invention for use in measurement of the concentration of at least one analyte contained in liquid sample by way of at least one of the optical measurement or image measurement comprises:
wettable materials that can be moistened with the liquid sample; and
a binding reagent put in the wettable materials,
   wherein the binding reagent has a property capable of taking on a coloration by reacting with a substance containing the analytes, and wherein the binding reagent is contained in the wettable materials in a uniform state.

Particularly preferred, the chromatographic strip includes a sheet-like solid support, and wettable materials disposed on the support,
wherein the wettable materials can be moistened with the liquid sample,
wherein the wettable materials comprises a test sample loading area, a labeling reagent holding layer, an at least one reactive layer and an absorbing layer, all formed in such a way as parallel on the surface of the support,
wherein the at least one reactive layer has the reaction areas, and
wherein the liquid sample passes through the test sample loading area, labeling reagent holding layer, at least one reactive layer and absorption layer successively in this order.

Thus, according to the structures as described in above, the performance of immuno-chromatographic quantitative measurements is allowed to be enhanced remarkably.

### Brief Description of Drawings

Fig. 1 is a schematic perspective view of a chromatographic quantitative measurement apparatus in an exemplary embodiment of the present invention.
Fig. 2 shows the steps of a chromatographic quantitative measurement method in an exemplary embodiment of the present invention.
Fig. 3 is a perspective view to show the structure of a chromatographic strip as used in the chromatographic quantitative measurement apparatus in an exemplary embodiment of the present invention.
Fig. 4 is a plan view of a chromatographic strip to show a pattern of reaction areas on at least one reactive layer in an exemplary embodiment of the present invention.
Fig. 5 is a plan view of a chromatographic strip to show another pattern of reaction areas on at least one reactive layer in an exemplary embodiment of the present invention.
Fig. 6 is a plan view to show the structure of a conventional chromatographic strip.

### Preferred Embodiments of the Invention

A chromatographic quantitative measurement apparatus in an exemplary embodiment of the present invention measures the concentration of at least one analyte contained in liquid sample by analyzing the result of a coloration reaction occurring on a chromatographic strip. The chromatographic strip is formed of a sheet-like solid support and a plurality of wettable materials that are laminated on the support and capable of getting moistened. The plurality of the wettable materials are disposed parallel on the surface of the support and comprise a test sample loading area, labeling reagent holding layer, at least one reactive layer and absorption layer.
At least one reactive layer has a reaction area immobilizing a binding reagent capable of forming a specific bond with the analytes contained in the liquid sample. The result of a coloration reaction occurring on the at least one reactive layer is analyzed for chromatographic quantitative measurement. The chromatographic quantitative measurement apparatus is provided with the chromatographic strip, a coloration level measuring means to measure quantitatively the extent of a coloration reaction occurring in the reaction areas by optical measurement or image measurement and a computation processing device to derive by computation the concentration of the analytes with a numerical expression by subjecting the measurement result to a computation process.

Accordingly, by conducting measurement at a plurality of locations in reaction areas on at least one reactive layer of a chromatographic strip, where a binding reagent capable of forming a bond with at least one analyte is immobilized, a coloration reaction on the test strip is allowed to be measured accurately without needing a control of infiltration conditions of liquid sample. As a result, the quantitative measurement performance is enhanced remarkably.

Particularly preferred is that the reaction areas are formed on the at least one reactive layer at a plurality of locations in a spots-like shape, respectively, in the chromatographic quantitative measurement apparatus. Reaction areas with the binding reagent immobilized thereon, each having a spots-like shape such as a dot or a ball, are disposed at a plurality of locations on the at least one reactive layer in irregular manner, thereby allowing the measurement of a coloration level to be conducted all over the surface of the at least one reactive layer by making a comparison in color between a reaction area taking on a coloration in a certain extent and another reaction area taking on a coloration in a different extent. Furthermore, since the reaction areas are disposed uniformly all over the at least one reactive layer in an irregular manner, the quantitative measurement can be carried out more accurately even when the coloration reaction is affected by infiltration conditions of the liquid sample. In addition, since the binding reagent is immobilized in a spots-like manner, the required amount of the reagent is reduced, resulting in a cost reduction.
Thus, a coloration reaction takes place without being affected by the infiltration condition of the liquid sample.

Particularly preferred is that the foregoing reaction areas with the binding reagent immobilized thereon are shaped like a flat plane, respectively, and disposed all over the reactive layer. According to this structure, the entire surface of the reactive layer acts as a reaction area, thereby allowing the coloration reaction to be measured across the whole surface of the reactive layer. Further, by having the reaction area disposed all over the reactive layer, even when the coloration reaction is affected by the infiltration conditions of the liquid sample, the quantitative measurement is allowed to be carried out more accurately. In other words, the coloration reaction takes place without being affected at all by the infiltration conditions of the liquid sample.

Particularly preferred is that the concentration of the binding reagent immobilized on the one reactive layer is to be kept consistent on the entire reaction areas, resulting in realizing a uniform coloration reaction. Because of the consistent concentration of the binding reagent immobilized on the reactive layer extending all over the reaction area, even when a coloration reaction takes place under an effect of infiltration conditions of the liquid sample, it is possible for the reaction conditions of the entire reactive layer to be fully grasped, thereby allowing the quantitative measurement to be carried out more accurately. Thus, the coloration reaction takes place without being affected by the infiltration conditions of the liquid sample.

Particularly preferred is that the reaction areas are disposed on re-active layer in a uniformly dispersed manner, thereby allowing a uniform coloration to be taken on. As a result, the accuracy of quantitative measurement is enhanced.

Chromatographic quantitative measurement method in an exemplary embodiment of the present invention comprises the steps of:
measuring by optical measurement or image measurement a plurality of locations in reaction areas where a coloration reaction is taking place, the reaction area being located on a chromatographic strip comprising layer, which is formed of materials ranging from one to an arbitrary number in kinds and capable of being moistened, and also being immobilized with a binding reagent capable of forming an specific bond with a substance containing at least one analyte included in liquid sample; and
applying a computation process to the measurement result to derive by computation the concentration of the analytes with numerical expression. Accordingly, without any control applied to moist conditions of the liquid sample, the coloration reaction occurring on the test strip is allowed to be measured accurately with resulting enhancement in the quantitative measurement performance.

Particularly preferred is that the reaction areas are formed on the at least one reactive layer at a plurality of locations in a spots-like shape, respectively. The configuration of each respective reaction area with a binding reagent immobilized thereon is formed in such spots-like shapes as dot and circles, and there are provided one or more of spots-like reaction areas located consistently in an irregular manner. As a result, the coloration reaction takes place without depending on the infiltration conditions of the liquid sample.

Particularly preferred is that the same binding reagent as immobilized on the at least one reactive layer is immobilized on all the reaction areas. In other words, there are provided the same binding reagents in such spots-like shapes as dots or balls disposed on each respective reaction area uniformly and yet in an irregular manner, thereby coloration reaction being taken on without depending on the moisten conditions of the liquid sample.

Particularly preferred is that the reaction areas are formed all over the surface of the at least one reactive layer. The reaction areas with the binding reagent immobilized thereon have a flat plane-like configuration, respectively, thereby allowing the existence of the reaction areas to be covering the at least one reactive layer extensively. As a result, a coloration reaction takes place without being controlled by the moist conditions of the liquid sample.

Particularly preferred is that the concentration of the binding reagent immobilized on the at least one reactive layer is consistent throughout the entire reaction areas, resulting in realizing a uniform coloration reaction.

Next, a detailed description is given to a chromatographic quantitative measurement apparatus and a method of using the apparatus in an exemplary embodiment of the present invention. The chromatographic quantitative measurement apparatus in the exemplary embodiment of the present invention features in improving the structure of an immuno-chromatographic strip and also in conducting optical measurement or image measurement on coloration reaction that is the result of a reaction between the analytes and the binding reagent contained in the chromatographic strip with the measurement result converted to a numerical expression by means of a computation processing device, thereby allowing the concentration of the analytes contained in the liquid sample to be derived quantitatively. In immuno-chromatographic quantitative measurement, an immuno-chromatographic strip having higher sensitivity and
more excellent performance in quantitative accuracy is made available and, therefore, the immuno-chromatographic strip features in having many reaction areas provided on the reactive carrier formed of a chromatographic material.

The chromatographic quantitative measurement apparatus with the method of using the apparatus of the present exemplary embodiment measures the extent of a coloration occurring on the immuno-chromatographic strip at a plurality of locations thereof by using measurement equipment, and then derives the concentration of the analytes by applying a computation process to the measurement result. Accordingly, a chromatographic quantitative measurement apparatus with high sensitivity and excellent performance and a measurement method using the apparatus, featuring insusceptibility to the flow rate and osmosis rate of a test sample on the chromatographic strip are allowed to be realized.

Each respective reaction area on a chromatographic strip has a binding reagent, which is allowed to form a specific bond with the analytes, immobilized thereon. This binding reagent has a consistent concentration throughout the entire reaction areas. By the use of a chromatographic strip of the foregoing structure, the reaction areas and measurement points are no longer needed to be restricted to a specific place in position, respectively, thereby allowing an arbitrary point of the reactive carrier to take on a coloration reaction. As a result, a coloration reaction reflecting the condition of dissolving of a labeling reagent, which takes place as the osmosis of the liquid sample proceeds, appears all over the reactive carrier. These degrees in coloration level are measured by using a coloration level measuring means and the measurement result is subjected to a computation process, thereby allowing higher sensitivity and higher performance chromatographic quantitative measurements to be realized.

With the present exemplary embodiment, as the reactive carrier is used a test strip formed of a chromatographic material that is comprised of an arbitrarily porous carrier such as nitrocellulose and glass fiber filter paper. This test strip is allowed to posses the ability to detect by analysis a specific substance and to perform the quantification thereof. As the liquid sample can be used, for example, such test samples as water, an aqueous solution, urine, blood, a bodily fluid, a solution with a solid and powder or a gas dissolved, and the like. Such tests as, for example, a urine test, pregnancy test, water quality test, feces test, soil analysis, food analysis and the like can be performed, thereby allowing the quantitative measurement of at least one analyte existing in an arbitrarily picked solution for testing to be conducted readily and reliably.

### Exemplary Embodiments

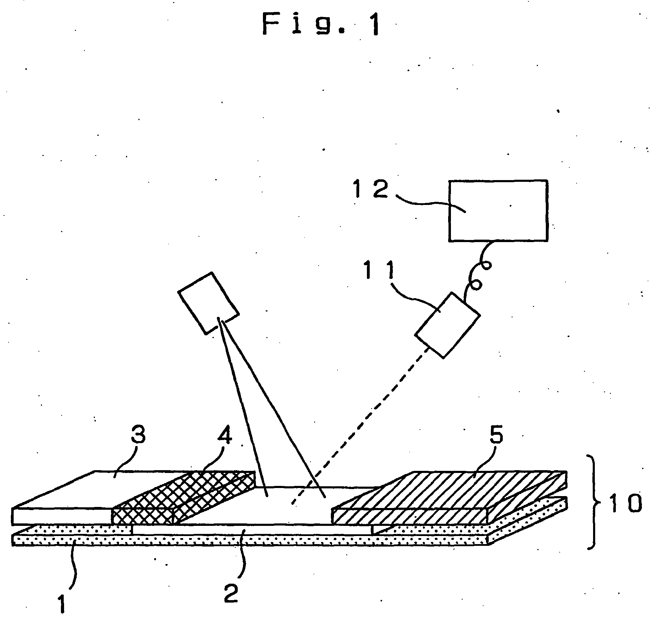

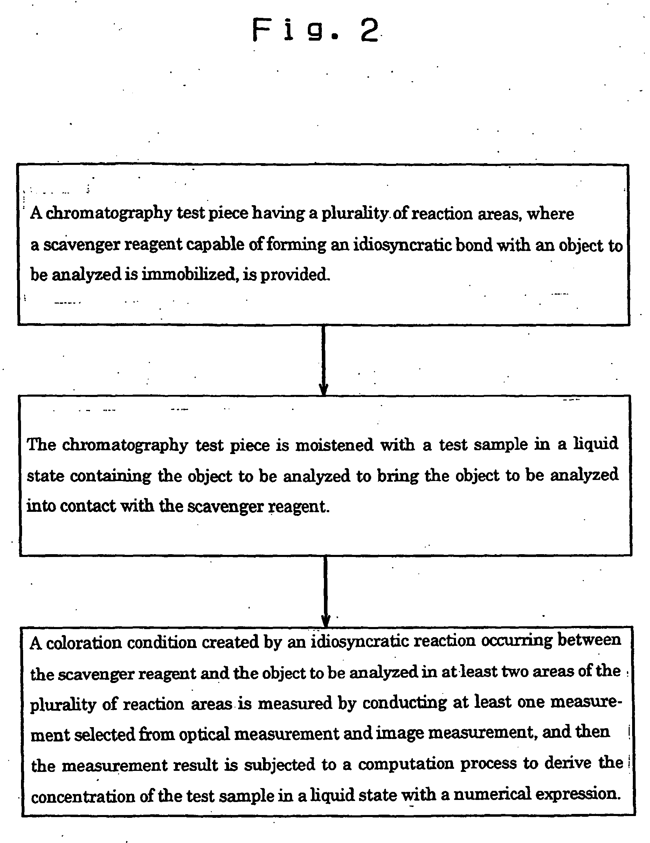

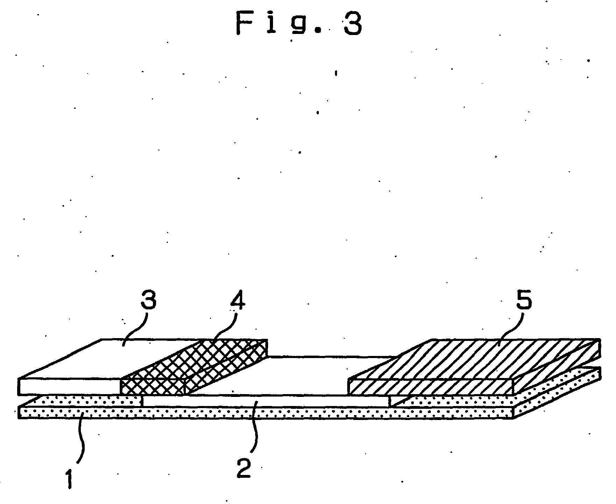

A description is given to a chromatographic quantitative measurement apparatus, measurement method using the foregoing apparatus and chromatographic strip in a typical exemplary embodiment of the present invention, respectively, with reference to Figures 1 to 5. Fig. 1 is a schematic perspective view of a chromatographic quantitative measurement apparatus in an exemplary embodiment of the present invention. Fig. 2 shows the steps of a chromatographic quantitative measurement method in an exemplary embodiment of the present invention. Fig. 3 is a perspective view to show the structure of an immuno-chromatographic strip as used in conducting immune measurement in an exemplary embodiment of the present invention. Here, the immuno-chromatographic strip is collectively called as reactive carrier.

As Fig. 2 shows, a chromatographic quantitative measurement method in a typical exemplary embodiment of the present invention for measuring the concentration of at least one analyte contained in liquid sample comprises the steps of:
(a) providing a chromatographic strip having a plurality of reaction areas with a binding reagent, which is capable of forming a bond with a substance containing the analytes, immobilized thereon;
(b) bringing the substance containing the analytes into contact with the binding reagent by moistening the chromatographic strip with the liquid sample containing the analytes; and
(c) measuring a coloration occurring due to a specific bond formed between the binding reagent and the analytes in at least two areas of the plurality of reaction areas by means of at least one method selected from the methods of optical measurement or image measurement and deriving by computation the concentration of the analytes in a numerical expression by subjecting the measurement result to computation processing.

As Fig. 3 shows, reactive 10 comprises a support 1, at least one reactive layer 2, test sample loading area 3, labeling reagent holding layer 4 and water absorption layer 5. The reactive layer is structured so as to have the liquid sample flown by passing through the test sample loading area 3, labeling reagent holding layer 4, at least one reactive layer 2 and water absorption layer 5 successively in this order.

The support 1 has a function of holding a chromatographic material and is formed of a plastic material and the like. The test sample loading area 3 is disposed on the support 1 and has a property of getting easily moistened with liquid sample. The test sample loading area 3 provided with the nature of becoming moist is formed of a nonwoven fabric and the like, for example. Liquid sample containing at least one analyte is added or applied by coating onto the test sample loading area 3. The labeling reagent holding layer 4 comprises wettable materials and a labeling reagent held by the moistening materials. The labeling reagent is made movable as the osmosis of the liquid sample takes place. The wettable materials are formed of a non-woven fabric and the like, for example. At least one reactive layer 2 is an area where a reaction between an antibody and an antigen occurs. At least one reactive layer 2 is formed of nitrocellulose and the like. At least one reactive layer 2 has a plurality of reaction areas, each of which has a binding reagent to form an specific bond with at least one analyte (a substance to be examined) immobilized thereon.
The plurality of reaction areas are disposed on at least one reactive layer 2 in such a way as situated uniformly or irregularly, dispersed uniformly or in a spots-like manner, or interspersed in dots. The water absorption layer 5 is an area where absorption of water from the liquid sample ultimately takes place.

With the chromatographic strip serving as at least one reactive layer carrier as describe in above, the liquid sample containing the analytes is added to the test sample loading area 3. When the liquid sample reaches the area of labeling reagent holding layer 4 from the test sample loading area 3, the labeling reagent held by the labeling reagent holding layer 4 is dissolved through the osmosis of the liquid sample. During this process, a bond between the analytes and the labeling reagent is being formed. When the substance resulting from the bond and containing the analytes flows to reach the at least one reactive layer 2, an specific chemical reaction occurs between the binding reagent contained in the reaction area and the flown analytes to cause a coloration reaction, resulting in generation of a coloration or a reaction between the binding reagent and at least one selected from the analytes and the labeling reagent occurs with resulting generation of a coloration. Ultimately, the liquid sample flows to reach the water absorption layer 5, where water is absorbed from the liquid sample. Alter the liquid sample is added to the at least one reactive layer carrier, the resulting reactive carrier is left standing for a predetermined period. By taking quantitative measurement of coloration that occurs after the lapse of the foregoing predetermined period, a quantitative measurement value of the substance to be examined can be obtained in a numerical expression.

More specifically, when liquid sample is added to the test sample loading area 3, the liquid sample reaches the labeling reagent holding layer 4 by osmosis through the test sample loading area 3. The labeling reagent is dissolved in the labeling reagent holding layer 4 by the liquid sample that arrives through osmosis and then proceeds to the at least one reactive layer 2 through osmosis. During this process, a bond between the analytes contained in the liquid sample and the labeling reagent is formed, thereby producing a substance resulting from the bond. This substance resulting from the bond causes a specific reaction with the binding reagent to occur on the at least one reactive layer 2, resulting in a coloration reaction. The liquid sample is ultimately absorbed in the water absorption layer 5, thereby bringing the entire reaction to an end.

When the analytes exists in the liquid sample, some coloration reaction is observed in the reaction area of the at least one reactive layer 2. This coloration reaction is measured by the use of a coloration level measuring means 11 such as optical measurement, image measurement and the like. The measurement result is subjected to computation processing, thereby the concentration of the analytes being derived by computation in a numerical expression.

Further, in the foregoing exemplary embodiment, the support 1 is formed of plastic and the at least one reactive layer 2, test sample loading area 3, labeling reagent holding layer 4 and water absorption layer 5 are formed of an unwoven fabric and nitrocellulose, but these materials are mentioned just for example and not for excluding other materials for a possible adoption. For example, the support 1 can be formed of an arbitrarily selected material that has a property of blocking the passing a liquid. Wettable materials for use in the at least one reactive layer 2, test sample loading area 3, labeling reagent holding layer 4, water absorption layer 5 and the like can be formed of an arbitrarily selected porous carrier.

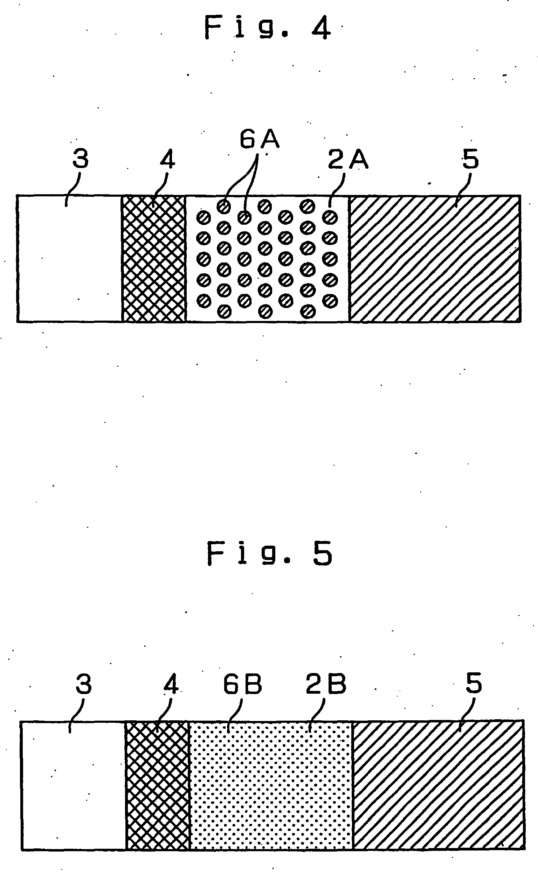

Next, a description is given to geometries of reaction areas of the at least one reactive layer 2 with reference to Fig. 4 and Fig. 5. Fig. 4 and Fig. 5 are a plan view of an example of reactive carrier 10, respectively, each having reaction areas different from one another. In Fig. 4, reactive layer 2A has reaction areas 6A formed of a plurality of dots or spots disposed thereon, each having a binding reagent immobilized thereto. In Fig. 5, reactive layer 2B has reaction areas 6B, to which a binding reagent is immobilized, disposed all over reactive layer 2B. By having reactive layer 2A or reactive layer 2B structured as described in above, the analytes that flows over the reactive carrier 10 can be bound without fail regardless of osmotic conditions. As a result, adverse effects due to a variation, inconsistency and the like existing among the test strips in coloration level are allowed to be prevented, thereby an accurate coloration being taken on.

In order to conduct measurements of a coloration level of the reactive layer 2 by the use of an immuno-chromatographic strip having the structure as described in above, a plurality of reaction areas randomly selected are sampled and measurements of the selected reaction areas are carried out.

As Fig. 1 shows, as the coloration level measuring means 11 can be used an instrument to read an absorption signal of a coloration, an instrument to read a reflecting signal of a coloration or an image analysis instrument using CCD.

In addition, as the computation processing device 12 is used an ordinary computation processing device. This computation processing device derives by computation the concentration of the analytes in a numerical expression based on the foregoing measurement result, thus allowing an average value of the coloration levels to be derived by computation. As the number of sampling is increased, the more accurate value in the quantitative measurement can be obtained.

Accordingly, a chromatographic quantitative measurement apparatus, which allows the quantitative measurement of high sensitivity and enhanced performance to be conducted, can be realized.

As described in above, with the measurement apparatus of the present invention, a provision of an optical measuring means or an image measuring means to measure a coloring extent in a reaction area allows the measurement of the coloration level to be conducted more accurately, hence enabling accurate quantitative measurement to be carried out by using a chromatographic strip.

By having a spots-like shaped reaction area disposed at a plurality of locations on reactive layer, the coloration level on all over the reactive layer is allowed to be measured accurately by making a comparison in coloration between the reaction area and the non-reaction area. In addition, the reaction areas are located on the at least one reactive layer in an irregular manner and therefore, even if a coloration reaction takes place under the influence of osmotic conditions of the liquid sample, more accurate quantitative measurement can be conducted. Furthermore, since the binding reagent is immobilized in a spots-like manner, the quantity of the reagent is allowed to be minimized, resulting in a cost reduction. Since the entire surface of the reactive layer acts as a reaction area, the coloration reaction occurring all over the reactive layer can be measured. At the same time, even when a coloration reaction influenced by osmotic conditions of the liquid sample occurs, more accurate quantitative measurement can be carried out because of the uniform reaction areas disposed all over the reactive layer.

According to the measurement method of the present invention, the condition of a coloration reaction occurring on each respective reaction area is measured at a plurality of locations on the at least one reactive layer and the measurement results are subjected to computation processing. Therefore, a good grasp of the coloration reaction conditions of the entire surface of the reactive layer is made possible, thereby enabling more accurate quantitative measurement to be conducted without receiving any influence of the osmotic conditions of the liquid sample.

Having a spots-like shaped reaction area formed at a plurality of places on the at least one reactive layer makes it possible for the coloration level measurement to be conducted by making a comparison between the reaction area and the area where no content of the binding reagent exists. Also, since the reaction areas are located on the at least one reactive layer in an irregular manner, even if a variation is created in the coloration conditions on the at least one reactive layer due to the influence of osmotic conditions of the liquid sample, the extent of the variation can be reduced by the computation processing after measurements performed at a plurality of locations on the at least one reactive layer, hence allowing the quantitative measurement to be carried out more accurately.

The kind of a binding reagent of each respective reaction area as disposed in a spots-like shape at a plurality of locations on the at least one reactive layer is the same throughout all the reaction area, thereby allowing the measurement of the entire reactive layer in coloration reaction to be carried out. At the same time, by making a comparison in coloration level between a reaction area and an area without any content of the binding reagent, the quantitative measurement can be performed more accurately. Further, since the reaction areas are located on the at least one reactive layer in an irregular manner, even if a variation is created in the coloration conditions on the at least one reactive layer due to the influence of osmotic conditions of the liquid sample, the extent of the variation can be reduced by the computation processing after measurements performed at a plurality of locations on the at least one reactive layer, hence allowing the quantitative measurement to be carried out more accurately. Additionally, since the binding reagent is immobilized in a spots-like manner, the quantity of the reagent is allowed to remain minimal, thereby making it possible to achieve a cost reduction. Since the entire surface of the at least one reactive layer acts as a reaction area, the coloration reaction occurring all over the at least one reactive layer becomes measurable. Further, since the reaction areas are disposed on the entire surface of the at least one reactive layer uniformly, even if a variation is created in the coloration conditions on the at least one reactive layer due to the influence of osmotic conditions of the liquid sample, the extent of the variation can be reduced by the computation processing after measurements performed at a plurality of locations on the at least one reactive layer, hence allowing the quantitative measurement to be carried out more accurately.

Since the concentration of the binding reagent immobilized on the at least one reactive layer is consistent throughout all the reaction areas, even if the coloration reactions are influenced by the osmotic conditions of the liquid sample, it becomes possible for the reaction conditions of the at least one reactive layer as a whole to be grasped. Further, even if a variation is created in the coloration conditions on the at least one reactive layer due to the influence of osmotic conditions of the liquid sample, the extent of the variation can be reduced by the computation processing after measurements performed at a plurality of locations on the at least one reactive layer, hence allowing the quantitative measurement to be carried out more accurately.

### Industrial Usability

According to the structure as disclosed by the present invention, any controlling of the osmosis of liquid sample and the development of a binding reagent is made no longer necessary, thereby making it possible for quantitative chromatographic measurements to be conducted with higher sensitivity and more excellent performance.

## Claims

1. A chromatographic quantitative measurement apparatus for measuring concentrations of at least one analyte contained in liquid sample, comprising:
a chromatographic strip with a plurality of reaction areas, each of said plurality of reaction areas retaining binding reagent that can take on coloration by a specific reaction with said at least one analyte; and
coloration level measuring means to conduct quantitative measurement with numerical expression on coloration level of at least two or more reaction areas out of said plurality of reaction areas,
wherein said coloration level measuring means have a function of conducting at least one measurement of an optical measurement and an image measurement of said coloration level of the coloration that occurs in the specific reaction between said binding reagent and said analytes.

2. The chromatographic quantitative measurement apparatus according to Claim 1, further comprising a computation processing device; said computation processing device having a computation process for processing measurement result of said coloration level and deriving the concentration of said analytes in the numerical expression.

3. The chromatographic quantitative measurement apparatus according to Claim 1,
wherein said chromatographic strip includes a sheet-like solid support and at least one reactive layer superimposed on said support,
wherein said reactive layer has said plurality of reaction areas disposed on said reactive layer uniformly;
wherein said reactive layer has wettable materials that can be moistened with said liquid sample;
wherein said binding reagent is contained in said wettable materials;
wherein said binding reagent reacts with substance containing said analytes to undergo coloration reaction; and
said coloration level measuring means to have coloration condition measured over said plurality of reaction areas disposed on said reaction layer uniformly.

4. The chromatographic quantitative measurement apparatus according to Claim 1,
wherein said chromatographic strip includes a sheet-like solid support, a, test sample loading area, a labeling reagent holding layer, at least one reactive layer and absorption layer,
wherein said labeling reagent holding layer, at least one reactive layer and absorption layer are, respectively, disposed on said support;
wherein said reactive layer has said plurality of reaction areas; and
wherein said liquid sample passes through said test sample loading area, said labeling reagent holding layer, said reactive layer and said absorption layer successively in this order.

5. The chromatographic quantitative measurement apparatus according to Claim 1,
wherein said plurality of reaction areas includes at least one reaction area of a plurality of dot-like areas and a plurality of spots-like shaped reaction areas; and
wherein said coloration level measuring means measures a coloration level of said at least one reaction areas.

6. The chromatographic quantitative measurement apparatus according to Claim 1,
wherein said chromatographic strip includes at least one reactive layer; and
wherein said pluralities of reaction areas are disposed all over said reactive layer.

7. The chromatographic quantitative measurement apparatus according to Claim 1,
wherein said chromatographic strip includes at least one reactive layer;
wherein said reactive layer has wettable materials to allow said liquid sample to be moistened;
wherein said binding reagent is contained in said wettable materials;
wherein said plurality of reaction areas are disposed all over said reactive layer; and
wherein a concentration of said binding reagent in said wettable materials are consistent in all areas of said plurality of reaction areas.

8. The chromatographic quantitative measurement apparatus according to Claim 1,
wherein said coloration level measuring means includes at least one device selected from the group consisting of a device to read an absorption signal corresponding to said coloration, a device to read a reflection signal of said coloration and an image analyzing device equipped with CCD.

9. The chromatographic quantitative measurement apparatus according to Claim 1,
wherein said chromatographic strip further includes a labeling reagent holding layer;
wherein said labeling reagent layer has labeling reagent capable of bonding with at least one of said analytes and said binding reagent; and
wherein said coloration occurs by bonding reaction between said binding reagent and said labeled reagent.

10. The chromatographic quantitative measurement apparatus according to Claim 1,
wherein said chromatographic strip includes a sheet-like solid support and a plurality of wettable materials disposed on said support;
wherein said plurality of wettable materials can be moistened with said liquid sample;
wherein said plurality of wettable materials have, respectively, a test sample loading area, labeling reagent holding layer, at least one reactive layer, and an absorption layer, which are formed parallel on a surface of said support;
wherein said reactive layer has said plurality of reaction areas; and
wherein said liquid sample passes through said test sample loading area, said labeling reagent holding layer, said reactive layer and said absorption layer successively in this order.

11. The chromatographic quantitative measurement apparatus according to Claim 10,
wherein said plurality of reaction areas have a plurality of spots; and
wherein said coloration level measuring means carries out quantitative measurement of the coloration level of said plurality of spots.

12. The chromatographic quantitative measurement apparatus according to Claim 10,
wherein said plurality of reaction areas are formed all over said reactive layer; and
wherein said coloration level measuring mean carries out quantitative measurement of the coloration level of said reactive layer.

13. The chromatographic quantitative measurement apparatus according to Claim 10,
wherein said plurality of reaction areas are formed all over said reactive layer;
wherein a concentration of said binding reagent is consistent all over said reactive layer; and
wherein said coloration level measuring means, carries out quantitative measurement of the coloration level of said reactive layer.

14. The chromatographic quantitative measurement apparatus according to Claim 10,
wherein said labeling reagent holding layer has labeling reagent capable of forming a bond with at least one selected from said analytes and said binding reagent; and
wherein said coloration occurs by bonding reactions between said binding reagent and said labeling reagent.

15. A method of chromatographic quantitative measurement to measure a concentration of at least one analyte contained in liquid sample, comprising the steps of:
(a) providing a chromatographic strip that has a plurality of reaction areas immobilizing a binding reagent capable of forming a specific bond with at least one analyte;
(b) moistening said chromatographic strip moistened with liquid sample containing said analytes, and bringing a substance containing said analytes into contact with said binding reagent; and
(c) measuring a condition of coloration in at least two reaction areas of said plurality of reaction areas, said coloration being taken on by specific bond between said binding reagent and said analytes, said coloration being measured by way of at least one measurement of an optical measurement and an image measurement, and deriving by computation a concentration in a numerical expression by subjecting a foregoing measurement result to computation processing.

16. The method of chromatographic quantitative measurement according to Claim 15,
wherein said chromatographic strip has wettable materials;
wherein said binding reagent is immobilized in said wettable materials; and
wherein in said step (b), while said liquid sample passes through said wettable materials, said substance containing said analytes reacts with said binding reagent, and said coloration occurs.

17. The method of chromatographic quantitative measurement according to Claim 15,
wherein said chromatographic strip has at least one reactive layer;
wherein said plurality of reaction areas are formed on said reactive layer in spots-like topology; and
wherein said condition of coloration of said spots-like topology is measured with the numerical expression in said step (c).

18. The method of chromatographic quantitative measurement according to Claim 15,
wherein each of said binding reagents contained in said plurality of reaction areas has identical binding reagent.

19. The method of chromatographic quantitative measurement according to Claim 15,
wherein said chromatographic strip has at least one reactive layer; and
wherein said plurality of reaction areas are formed all over said reactive layer.

20. The method of chromatographic quantitative measurement according to Claim 15,
wherein said chromatographic strip has wettable materials;
wherein said binding reagent is immobilized in said wettable materials; and
wherein a concentration of said binding reagent in said wettable materials is identical throughout all of said plurality of reaction areas.

21. The method of chromatographic quantitative measurement according to Claim 15,
wherein said chromatographic strip has a sheet-like solid support and a plurality of wettable materials disposed on said support;
wherein said plurality of wettable materials can be moistened with said liquid sample;
wherein said plurality of wettable materials have, respectively, a test sample loading area, a labeling reagent holding layer, at least one reactive layer and an absorption layer that are all disposed parallel on a surface of said support;
wherein said reactive layer has said plurality of reaction areas;
wherein said liquid sample passes through said test sample loading area, said labeling reagent holding layer, said reactive layer and said absorption layer successively in this order;
wherein said labeling reagent holding layer has labeling reagent capable of forming bond with at least one selected from said analytes and said binding reagent; and
wherein said coloration occurs by bonding reaction occurring between said binding reagent and said labeling reagent.

22. A chromatographic strip for measuring a concentration of at least one analyte contained in liquid sample by conducting at least one measurement selected from optical measurement and image measurement, comprising:
wettable materials that can be moistened with liquid sample; and
binding reagent put in said wettable materials,
wherein said binding reagent has a nature capable of taking on a coloration as a result of specific reaction with substance that contains said analytes; and
wherein said binding reagent is contained in said wettable materials uniformly.

23. The chromatographic strip according to Claim 22,
wherein said wettable materials has a test sample loading area, a labeling reagent holding layer, at least one reactive layer and an absorption layer;
wherein said reactive layer has a plurality of reaction areas, each of said plurality of reaction areas containing said binding reagent; and
wherein said liquid sample is allowed to pass through said test sample loading area, said labeling reagent holding layer, said reactive layer and said absorption layer successively in this order.

24. The chromatographic strip according to Claim 23,
wherein said plurality of reaction areas have a plurality of spots; and
wherein coloration level of each of said plurality of spots is allowed to be measured quantitatively by conducting at least one measurement of said optical measurement and said image measurement.

25. The chromatographic strip according to Claim 23,
wherein said wettable materials has at least one reactive layer; and
wherein said plurality of reaction areas are formed all over said reactive layer.

26. The chromatographic strip according to Claim 23,
wherein a concentration of said binding reagent in said wettable materials is consistent throughout all areas of said plurality of reaction areas.

27. The chromatographic strip according to Claim 22,
wherein said plurality of wettable materials can be moistened with said liquid sample;
wherein said plurality of wettable materials have, respectively, a test sample loading area, labeling reagent holding layer, at least one reactive layer and absorption layer that are all formed parallel on a surface of said support;
wherein said reactive layer has said plurality of reaction areas;
wherein said liquid sample passes through said test sample loading area, said labeling reagent holding layer, said reactive layer and said absorption layer successively in this order; and
wherein said labeling reagent holding layer has labeling reagent capable of forming bond with at least one selected from said analytes and said binding reagent, and said coloration occurs by bonding reaction occurring between said binding reagent and said labeling reagent.

28. The chromatographic strip according to Claim 23,
wherein said plurality of reaction areas is disposed all over said reactive layer in a uniformly dispersed manner.
